# FASCICULE DE BREVET EUROPEEN

(11) **EP 1 118 349 B1**
(45) Date de publication et mention de la délivrance du brevet: **12.01.2005**
(21) Numéro de dépôt: 01400152.3
(22) Date de dépôt: 19.01.2001
(51) Int. Cl.: A61N 1/05

(54) **Sonde monocorps pour dispositif médical implantable actif de type défibrillateur/cardioverteur implantable**
Einteilige Sonde für medizinische aktive Vorrichtungen wie implantierbarer Defibrillator/Kardiovertierer
One-piece probe for medical active device as implantable defibrillator/cardioverter

(30) Priorité: 19.01.2000 FR 0000632
(43) Date de publication de la demande: 25.07.2001
(73) Titulaire: ELA MEDICAL (Société anonyme), 92541 Montrouge (FR)
(72) Inventeur: Ripart, Alain, 91190 Gif sur Yvette (FR)
(74) Mandataire: Dupuis-Latour, Dominique

(56) Documents cités:
- DE-A- 19 800 697
- US-A- 4 499 907
- US-A- 5 713 945

## Description

L'invention concerne les "dispositifs médicaux implantables actifs" tels que définis par la directive 90/385/CEE du 20 juin 1990 du Conseil des communautés européennes.

Elle concerne plus particulièrement la famille des appareils chargés de délivrer au coeur des impulsions électriques de haute énergie (c'est-à-dire dépassant notablement l'énergie fournie pour la simple stimulation) pour tenter de mettre fin à une tachyarythmie. Ces dispositifs sont appelés "défibrillateurs implantables" ou " cardioverteurs implantables", étant entendu que l'invention couvre aussi bien les défibrillateurs/cardioverteurs/stimulateurs ou les défibrillateurs/stimulateurs implantables.

Ces dispositifs sont constitués de deux ensembles, à savoir un générateur d'impulsions et une sonde ou un système de sondes.

Le générateur d'impulsions est chargé de surveiller l'activité cardiaque et de générer des impulsions de haute énergie quand le coeur présente une arythmie ventriculaire susceptible d'être traitée. Quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation".

À ce générateur sont connectées une ou plusieurs sondes munie(s) d'électrodes dont le rôle est de distribuer au coeur cette énergie de façon appropriée. Le EP-A-0 773 039 (ELA Médical) décrit un tel ensemble générateur/sondes avec des moyens permettant de choisir la configuration optimale d'application du choc de cardioversion ou de défibrillation.

L'invention concerne le cas particulier où le générateur est relié à une sonde dite "monocorps", qui est une sonde unique portant les différentes électrodes permettant de délivrer le choc de défibrillation ou de cardioversion.

Le DE-A-198 00 697 (Biotronik) décrit une telle sonde monocorps comprenant deux électrodes ventriculaires de recueil d'un signal cardiaque, une électrode ventriculaire de choc, une électrode auriculaire de recueil d'un signal cardiaque et une électrode auriculaire de choc. Les deux électrodes auriculaires (détection et choc) sont reliées électriquement ensemble, ce qui permet de faire l'économie d'un conducteur dans la sonde (quatre conducteurs au lieu de cinq comme cela est le cas dans d'autres configurations de sonde, telle que celle illustrée par le US-A-5 713 945 (Pacesetter)) et également d'une borne de connexion du défibrillateur. En réalité, l'électrode de choc "auriculaire" est une électrode positionnée principalement dans la veine cave supérieure et, de ce fait, elle ne permet pas le recueil convenable d'un signal cardiaque ; c'est pour cette raison qu'il est prévu une électrode auriculaire de détection, placée effectivement dans l'oreillette, pour pouvoir recueillir l'activité électrique présente au sein du myocarde en cet endroit. Mais la mesure de l'activité auriculaire est opérée entre cette électrode auriculaire de recueil et la masse (mesure en mode monopolaire), ce qui conduit à une dégradation notable du rapport signal/bruit, la sonde étant une sonde flottante.

L'un des buts de l'invention est de remédier à cet inconvénient, en proposant une structure de sonde perfectionnée qui, sans augmenter le nombre de conducteurs et de bornes de connexion au générateur, procure une mesure de meilleure qualité de l'activité électrique dans l'oreillette.

La sonde de l'invention est du type général connu du DE-A-198 00 697 précité, à savoir comprenant un corps de sonde comportant une première électrode auriculaire de recueil d'un signal cardiaque auriculaire, une première et une seconde électrodes ventriculaires de recueil d'un signal cardiaque, une électrode ventriculaire d'application d'une énergie de défibrillation ou de cardioversion, et une électrode supraventriculaire d'application de ladite énergie de défibrillation ou de cardioversion, une liaison électrique, de préférence interne, étant prévue entre ladite première électrode auriculaire et l'électrode supraventriculaire.

De façon caractéristique de l'invention, la sonde comprend en outre une seconde électrode auriculaire de recueil d'un signal cardiaque auriculaire, et ladite électrode ventriculaire d'application d'une énergie de défibrillation ou de cardioversion est l'une desdites électrodes ventriculaires de recueil d'un signal cardiaque, ledit signal cardiaque auriculaire étant recueilli par la sonde entre, d'une part, l'ensemble constitué par l'électrode supraventriculaire et l'électrode auriculaire qui lui est reliée et, d'autre part, l'autre électrode auriculaire.

L'électrode auriculaire reliée à l'électrode supraventriculaire peut notamment être une électrode proximale, l'autre électrode auriculaire étant une électrode distale.

On va maintenant décrire un exemple de mise en oeuvre de l'invention en référence à la figure unique annexée, qui est une représentation schématique du muscle cardiaque avec une sonde monocorps implantée.

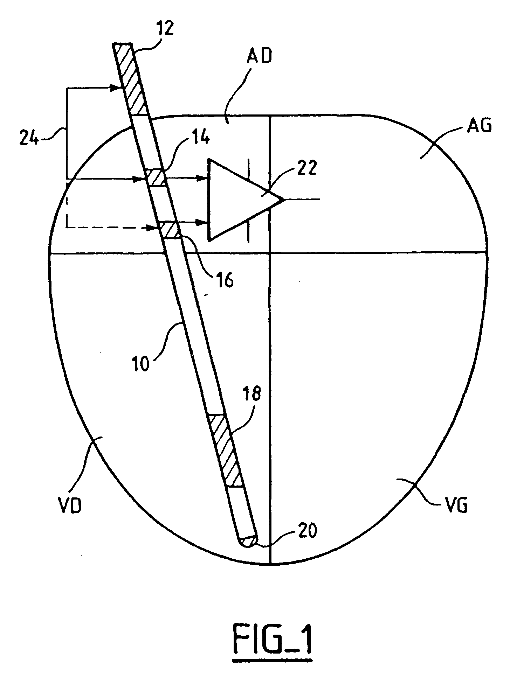

Sur la figure 1, on a représenté schématiquement le muscle cardiaque avec ses quatre cavités : oreillette droite AD, ventricule droit VD, oreillette gauche AG et ventricule gauche VG.

Une sonde monocorps 10 a été introduite par le réseau veineux dans les deux cavités auriculaire et ventriculaire, de manière à y détecter l'activité cardiaque et appliquer en tant que de besoin un choc de défibrillation ou de cardioversion.

Cette sonde monocorps 10 comporte, dans le sens proximal vers distal :
- une première électrode de choc 12, constituant par exemple la borne positive d'application du potentiel de défibrillation ou de cardioversion. Cette électrode est une électrode dite "supraventriculaire", typiquement une électrode bobinée positionnée principalement dans la veine cave supérieure,
- une électrode auriculaire proximale 14,
- une électrode auriculaire distale 16,
- une deuxième électrode de choc 18 située dans le ventricule, constituant par exemple la borne négative d'application du potentiel de défibrillation ou de cardioversion, et,
- une électrode ventriculaire d'extrémité 20.

De façon caractéristique de l'invention, l'activité auriculaire est détectée en mode bipolaire par une mesure différentielle opérée entre les électrodes 14 et 16 (mesure différentielle schématisée par l'amplificateur opérationnel 22).

Par ailleurs, la sonde comporte une liaison interne, schématisée en 24, entre l'électrode supraventriculaire 12 et l'électrode auriculaire proximale 14. Cette liaison réalise sur l'entrée correspondante de l'amplificateur 22 la sommation des signaux présents sur les deux électrodes ; du fait de sa position extérieure à l'oreillette, l'électrode supraventriculaire 12 ne recueille pas de signal cardiaque. Les électrodes 12 et 14 sont électriquement reliées et les signaux présents en 14 et 16 sont appliqués aux entrées de l'amplificateur différentiel 22. Celui-ci doit avoir un très bon taux de réjection ce qui permet la programmation d'une forte sensibilité, par exemple de 0,15 mV : une telle valeur est nécessaire pour détecter les signaux auriculaires à partir d'un montage flottant. En effet, aucune des électrodes n'est en contact avec le tissu cardiaque.

En variante, au lieu d'une liaison interne, la liaison 24 peut être réalisée par le connecteur du défibrillateur, mais cette variante implique la présence d'un conducteur supplémentaire dans la sonde.

En variante également, l'électrode supraventriculaire 12 est reliée à l'électrode auriculaire distale 16 au lieu de l'être à l'électrode auriculaire proximale 14, pour une meilleure réjection du mode commun par effet de blindage autour de l'électrode 14.

Les électrodes ventriculaires 18 et 20 peuvent également servir au recueil d'un signal ventriculaire : le défibrillateur opère ainsi en mode double chambre pour la détection (détection à la fois dans l'oreillette et dans le ventricule), avec application du choc de défibrillation et de cardioversion dans le ventricule seul, entre l'électrode ventriculaire 18 et l'électrode supraventriculaire 12. Un tel fonctionnement s'apparente, *mutatis mutandis*, à celui d'un dispositif VDD pour un stimulateur cardiaque.

## Revendications

1. Une sonde monocorps pour dispositif médical implantable actif de type défibrillateur/cardioverteur implantable, avec un corps de sonde (10) comportant :
- une première (14) électrode auriculaire de recueil d'un signal cardiaque auriculaire,
- une première (18) et une seconde (20) électrodes ventriculaires de recueil d'un signal cardiaque,
- une électrode ventriculaire d'application d'une énergie de défibrillation ou de cardioversion, et
- une électrode supraventriculaire (12) d'application de ladite énergie de défibrillation ou de cardioversion, une liaison électrique (24) étant prévue entre ladite première électrode auriculaire et l'électrode supraventriculaire (12),
sonde **caractérisée en ce qu'**elle comprend en outre une seconde électrode auriculaire (16) de recueil d'un signal cardiaque auriculaire, et **en ce que** ladite électrode ventriculaire d'application d'une énergie de défibrillation ou de cardioversion est l'une (18) desdites électrodes ventriculaires de recueil d'un signal cardiaque, ledit signal cardiaque auriculaire étant recueilli par la sonde entre, d'une part, l'ensemble constitué par l'électrode supraventriculaire (12) et l'électrode auriculaire (14 ; 16) qui lui est reliée et, d'autre part, l'autre électrode auriculaire (16 ; 14).

2. La sonde monocorps de la revendication 1, dans laquelle la liaison électrique (24) entre l'une des électrodes auriculaires et l'électrode supraventriculaire (12) est une liaison interne à la sonde.

3. La sonde monocorps de la revendication 1, dans laquelle l'électrode auriculaire (14) reliée à l'électrode supraventriculaire est une électrode proximale et l'autre électrode auriculaire (16) est une électrode distale.

## Claims

1. A single-body lead for an active implantable medical device of the implantable defibrillator/cardioverter type, having a lead body (10) comprising:
- a first (14) atrial electrode for sensing an atrial cardiac signal;
- a first (18) and a second (20) ventricular electrode for sensing a cardiac signal;
- a ventricular electrode for applying a defibrillation or cardioversion energy;
- a supra-ventricular electrode (12) for applying said defibrillation or cardioversion energy, an electrical connection (24) being provided between said first atrial electrode and the supra-ventricular electrode;
said lead being **characterised by** further comprising a second atrial electrode (16) for sensing an atrial cardiac signal, and in that said ventricular electrode for applying a defibrillation or cardioversion energy is one (18) of said ventricular electrodes for sensing a cardiac signal, said atrial cardiac signal being sensed by the lead between, on the one hand, the supra-ventricular electrode (12) and the atrial electrode (14 ; 16) connected thereto taken as a whole, and, on the other hand, the other atrial electrode (16 ; 14).

2. The single-body lead of claim 1, wherein the electric connection (24) between one of the atrial electrodes and the supra-ventricular electrode is a connection internal to the lead.

3. The single-body lead of claim 1, wherein the atrial electrode (14) connected to the supra-ventricular electrode is a proximal electrode and the other atrial electrode (16) is a distal electrode.

## Patentansprüche

1. Einteilige Sonde für eine aktive implantierbare medizinische Vorrichtung vom Typ implantierbarer Defibrillator/Kardiovertierer, mit einem Sondenkörper (10), der aufweist:
- eine erste (14) atriale Elektrode zum Empfang eines atrialen Herzsignals,
- eine erste (18) und eine zweite (20) ventrikuläre Elektrode zum Empfang eines Herzsignals,
- eine ventrikuläre Elektrode zur Abgabe einer Energie zur Defibrillation oder zur Kardioversion, und
- eine supraventrikuläre Elektrode (12) zur Abgabe der Energie zur Defibrillation oder zur Kardioversion, eine elektrische Verbindung (24), die zwischen der ersten atrialen Elektrode und der supraventrikulären Elektrode (12) vorgesehen ist,
wobei die Sonde **dadurch gekennzeichnet ist, dass** sie ferner eine zweite atriale Elektrode (16) zum Empfang eines atrialen Herzsignals umfasst, und dadurch, dass die ventrikuläre Elektrode zur Abgabe einer Energie zur Defibrillation oder zur Kardioversion eine (18) der ventrikulären Elektroden zum Empfang eines Herzsignals ist, wobei das atriale Herzsignal empfangen wird durch die Sonde zwischen, einerseits, der Gesamtheit, die durch die supraventrikuläre Elektrode (12) und die atriale Elektrode (14; 16) gebildet wird, die mit ihr verbunden ist, und, andererseits, der anderen atrialen Elektrode (16; 14).

2. Einteilige Sonde nach Anspruch 1, in welcher die elektrische Verbindung (24) zwischen einer der atrialen Elektroden und der supraventrikulären Elektrode (12) eine interne Verbindung der Sonde ist.

3. Einteilige Sonde nach Anspruch 1, in welcher die atriale Elektrode (14), die mit der supraventrikulären Elektrode verbunden ist, eine proximale Elektrode ist und die andere atriale Elektrode (16) eine distale Elektrode ist.
